# EUROPEAN PATENT SPECIFICATION

(11) **EP 2 120 765 B1**
(45) Date of publication and mention of the grant of the patent: **02.10.2013**
(21) Application number: 07849327.7
(22) Date of filing: 04.12.2007
(51) Int. Cl.: A61B 18/20, A61N 5/06

(54) **LED DEVICE FOR THE HAEMOSTASIS OF BLOOD VESSELS**
LED-VORRICHTUNG ZUR HÄMOSTASE VON BLUTGEFÄSSEN
DISPOSITIF LED POUR L'HÉMOSTASE DE VAISSEAUX SANGUINS

(30) Priority: 05.12.2006 IT FI20060307
(43) Date of publication of application: 25.11.2009
(73) Proprietor: Light 4 Tech Firenze S.r.l., 50018 Scandicci (FI) (IT)
(72) Inventor: PINI, Roberto, 50029 Impruneta (firenze) (IT); ROSSI, Francesca, 50019 Sesto Fiorentino (firenze) (IT)
(74) Representative: Brazzini, Silvia
(86) International application number: PCT/IB2007/054912
(87) International publication number: WO 2008/068712

(56) References cited:
- EP-A2- 1 410 768
- WO-A-01/08579
- WO-A-01/87176
- WO-A1-2006/098719
- GB-A- 2 360 461
- US-A- 5 634 711
- US-A1- 2003 233 138
- US-A1- 2005 152 146

## Description

### Field of the invention

The present invention relates generally to the sector of LED technologies used for the haemostasis of cutaneous blood vessels and its application to dermoplastic surgery.

In particular, the invention refers to a LED device for dealing with problems of bleeding from cutaneous capillary vessels.

The present invention also relates to a device of the aforesaid type for use in combination with a dermal ablation laser to reduce the side-effects of said treatment.

### State of the art

The haemostasis of blood vessels, and particularly of those of small calibre such as the superficial cutaneous capillaries, is currently achieved mainly in three ways: 1) by local mechanical compression, which interrupts the blood flow in the vessel, enabling coagulation to occur through platelet aggregation; 2) by pharmacological treatment, e.g. using a drug having a sclerosing or vasoconstrictor action; and 3) by a thermal coagulation inducing processes.

With particular reference to these processes, two classes of device can be identified that are used to obtain two distinct types of treatment, i.e. electrocoagulation and photocoagulation. In both cases, the increase in temperature induced in the tissue gives rise to a thermal denaturation of the proteins contained in the blood and in the structural component of the vessel, with a consequent haemostasis. Electrocoagulation is typically achieved using devices, such as diathermal coagulators - clamping devices that generate a highfrequency alternating current (higher than 300 kHz), which produces heat when applied to the tissues due to the Joule effect. The risks related with the use of this technology are high, however, because the thermal process that they induce is non-selective, i.e. it is impossible to restrict its action to the vessel requiring coagulation without heating the surrounding tissues too.

The term "photocoagulation" is used to mean a much more recent technique that is much less widespread than the previous one, which consists in irradiating the tissue with light energy that is subsequently converted into thermal energy, thereby inducing coagulation of the vessel.

This result is achieved using laser devices with a wavelengths typically in the near- or medium-infrared regions (0,7-10 µm), that are absorbed mainly by the water content of the tissues. These devices are essentially diode, erbium, neodymium and CO₂ lasers, that are already used in dermoplastic and angioplastic surgery. These lasers generally do not have a selective action on the haematic component without affecting the surrounding tissue.

Another type of laser has also recently been recommended for use in dermatology, i.e. the dye laser, with an emission around 580-590 nm, that has a selective sclerosing action on the larger-diameter cutaneous vessels (around 1 mm) and is used to remove superficial angiomas. These devices do not have such a selective action on the smaller-calibre cutaneous capillary vessels (10-100 microns), however, because the pulsed emission mode of the dye laser makes it impossible for the heat to build up enough to induce their coagulation. Moreover, these laser devices are still more expensive than those operating with wavelengths in the near- and medium infrared range and also demand very frequent maintenance, consisting in the replacement of the dye constituting the laser medium.

With particular reference to dermal ablation in cosmetic surgery, which consists in the removal of the epidermis and a part of the dermis, either by means of a laser or using mechanical instruments, to obtain an ablation of wrinkles (skin resurfacing), this ablation treatment very often causes bleeding from the cutaneous vessels of the dermal papilla (which are less than 100 micron in diameter). The bleeding occurring during the treatment is disadvantageous both for the patient and the surgeon, because it makes it impossible for the physician to have a clear view of the area being treated, it can cause infection and - in the case of laser dermal ablation - it acts as a screen against the laser radiation. When bleeding occurs, it becomes necessary to temporarily stop the treatment until the bleeding has ceased, which can take several minutes, and this means that the treatment session continuously stops and starts, with a significant increase in the overall operating times, causing discomfort to the patient and a greater cost of the treatment. Moreover, the resulting cosmetic effect may be invalidated by the presence of wide scarring areas.

In the field of dermoplastic surgery, there are basically two technical solutions currently proposed to overcome the problem of haemorrhage: 1) mechanical compression of the bleeding skin surface, which is the most straightforward solution, preferred in the majority of cases; 2) CO₂ laser irradiation, especially when the same laser is also used for the dermal ablation treatment. As already mentioned, however, the CO₂ laser is not selective for the haematic component, but induces coagulation of all the dermal tissue and often has an excessive thermal effect and consequently causes damage due to tissue burns, with consequent scarring complications.

In dermoplastic surgery, and more generally in the treatment of abrasive lesions, the problem of the bleeding control is therefore still an open issue, which has yet to find a simple and inexpensive solution. It is consequently of fundamental importance to provide a safe device that selectively affects the haematic component and that is inexpensive, manageable and user-friendly, possibly also designed for use in association with dermal ablation lasers (Erbium:YAG and CO₂) in cosmetic surgery, that enables bleeding side-effects to be rapidly and effectively overcome.

US 2003/0233138 discloses a device equipped with light emitting diodes (LEDs) for dermatological treatments in which a portion of the divergent light emitted by the the LEDs is the concentrated with an optical train and is trasmitted to a target region of the target tissue, the target region being smaller than the LED region, i.e. the area wherein the LEDs are distributed, so that the concentrated light selectively heats and treats the target tissue.

The LEDs used in this device may have various emission ranges according to the application including that of the blue light. Other devices for phototherapy are known from US 5634711 and GB 2360461.

### Summary of the invention

The general object of the present invention is to provide a small, manageable and portable LED device for inducing the haemostasis of cutaneous blood vessels by means of a photo-thermocoagulation process, i.e. using radiation at a wavelength selectively absorbed by the blood with a controlled heat release. The device is intended for dermal ablation treatments in cosmetic surgery and, more in general, in the treatment of superficial skin lesions howsoever induced, be it surgically or accidentally.

A particular object of the present invention is to provide an LED device that can be operated manually by direct application in contact with the skin, or focused by a suitable optical device, or transmitted using fibre-optic means, to induce haemostasis in cases of superficial vessel bleeding.

Another particular object of the present invention is to provide an LED device with a light emission associated with the handpiece of a laser used in surgical or cosmetic treatments, e.g. for dermal ablation procedures, with a view to inducing the haemostasis of superficial vessels during said procedures.

These objects are achieved with the LED device according to the present invention the essential features of which are set forth in the attached claim 1.

Further important features of the invention are described in the dependent claims.

### Brief description of the drawings

The characteristics and advantages of the haemostatic LED device will be apparent from the following description of an embodiment, given here as a non-limiting example with reference to the attached drawings, wherein:
figure 1 is a schematic view of the haemostatic LED device;
figure 2 shows how the haemostatic LED device according to the invention is used in association with a dermal ablation laser;
figure 3 shows the absorption spectrum of the main chromophores in the skin;
figure 4 shows the calculated temperature increase in a capillary due to the effect of blue light in the event of the LED device being used in contact with the skin;
figure 5 shows the calculated temperature increase in the thickness of the skin due to the effect of blue light produced by the same treatment as in figure 4;
figure 6 shows the temperature calculated in a capillary due to the effect of blue light in the event of the LED device being focused on the skin;
figure 7 shows the calculated temperature increase in the thickness of the skin due to the effect of blue light produced by the same treatment as in figure 6;
figure 8 shows the temperature calculated in a capillary due to the effect of blue light in the event of the LED device adopting fibre-optic means for transmitting the focused light emission;
figure 9 shows the calculated temperature increase in the thickness of the skin due to the effect of blue light produced by the same treatment as in figure 8.

### Detailed description of the invention

The LED device for the haemostasis of cutaneous blood vessels is based on the use of radiation in the blue-violet spectral band (390-470 nm) to induce coagulation of the vessels as a result of a photo-thermal effect. The LED device is designed to be used in direct contact with tissue, or focused using a suitable optical device, or using a fibre-optic transmission means which enables its use alone or in association with the handpiece of a laser or a high-power non-coherent light source configured for surgical or cosmetic treatments.

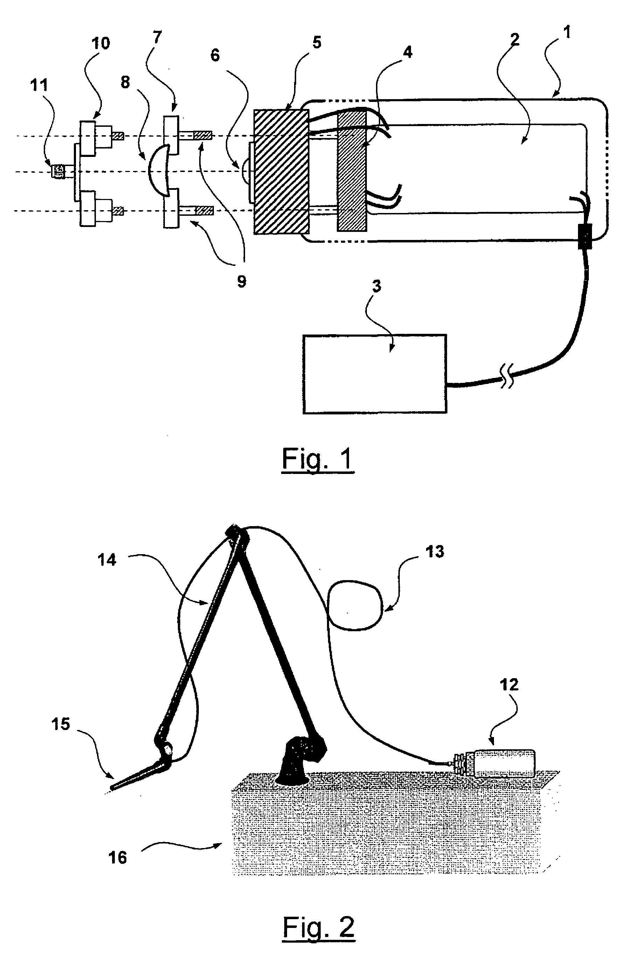

Figure 1 schematically shows an example of an LED device, where the numeral 1 indicates a cylindrical container sized to make it readily manageable, e.g. 5 cm in diameter by 12 cm in length. The container 1 houses a power supply unit 2 for an LED 6, e.g. of the analogic type with a stabilised voltage and current limiting means, powered at 24 V to reduce the risk of electric shock to either the patient or the operator. The power supply unit 2 is electrically connected to an external power supply 3 operating at the mains voltage that provides the 24 V power supply. The numeral 4 indicates a fan for cooling the LED 6, also powered by the aforesaid stabilized power supply unit 2. The LED 6 is installed on and thermally in contact with a heat sink 5 for dissipating the heat produced by the LED.

The numeral 7 identifies an optical system for focusing the light emission from the LED 6, comprising at least one lens 8; said optical system is easily attachable to the heat sink 5 by means of suitable spacers 9.

For the fibre-optic transmission of the light from the LED, if any, the device may also be fitted with fibre-optic coupling means 10 for installing on the aforesaid focusing system 7; the fibre is connected by means of a female connector 11, e.g. of the SMA screw type.

Figure 2 shows how the LED device 12 is used in association with a surgical or cosmetic laser device 16, where 13 is the optic fibre (e.g. 1 mm in diameter), 14 is the articulated arm of the laser and 15 is the handpiece on said articulated arm. The end of the optic fibre is locked in the handpiece so that the light from the LED is emitted coaxially to the light from the laser. Inside the handpiece, there may also be a suitable optical system, consisting for instance of optics for focusing the light emitted through the fibre and a dichroic reflector being transparent to the wavelength of the LED and completely reflecting that of the laser. The optical system replaces the last mirror of the articulated arm located immediately before the handpiece, so that the focal spot of the LED radiation overlaps with that of the laser radiation on the surface of the tissue being treated.

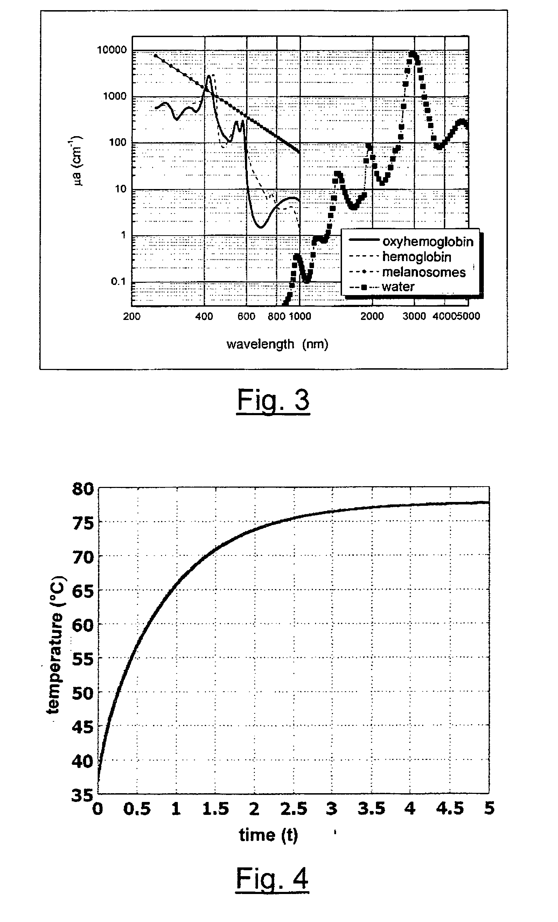

The LED component is in the form of a matrix of individual LEDs emitting light in the blue-violet band with a globally moderate-to-high power around 100 mW - 1W, such as those developed more recently and relatively inexpensively (see, for instance, the Roithner Lasertechnik GmbH catalogue). The choice of LED emission in the blue-violet spectral band is prompted by the fact that the principal optical absorption peak of oxygenated haemoglobin is located around 410 nm, while for non-oxygenated haemoglobin it is around 430 nm. The absorption of these two species in said spectral regions is considerably higher than that of other cutaneous components such as water and melanin, as shown in figure 3. The optical properties of haemoglobin in the blue spectral band can be exploited to induce photocoagulation in the dermal vessels of small-to-medium calibre (10-100 micron in diameter) in the event of losses due to exfoliation or abrasion of the epidermis. The limited depth of penetration of blue light in the dermal layer (to a few hundred microns) restricts the application of the device to the superficial capillary vessels.

Contact application method. In one possible embodiment, a light source comprises a LED array, e.g. the LED 435-66-60 (Roithner Lasertechnik, Vienna, Austria) which emits an average light power of 700 mW in a band coming between 430 and 440 nm, that is, in the vicinity of the absorption peak of non-oxygenated haemoglobin. The haemostatic treatment is achieved by placing the light emitting surface of the LED in direct contact with the cutaneous area that is bleeding. Said application method is suitable for the haemostasis of relatively wide areas of skin, i.e. around 1 cm². A sheet of sterile material transparent to the wavelength emitted by the device can be inserted between the skin and the light emitting surface of the LED.

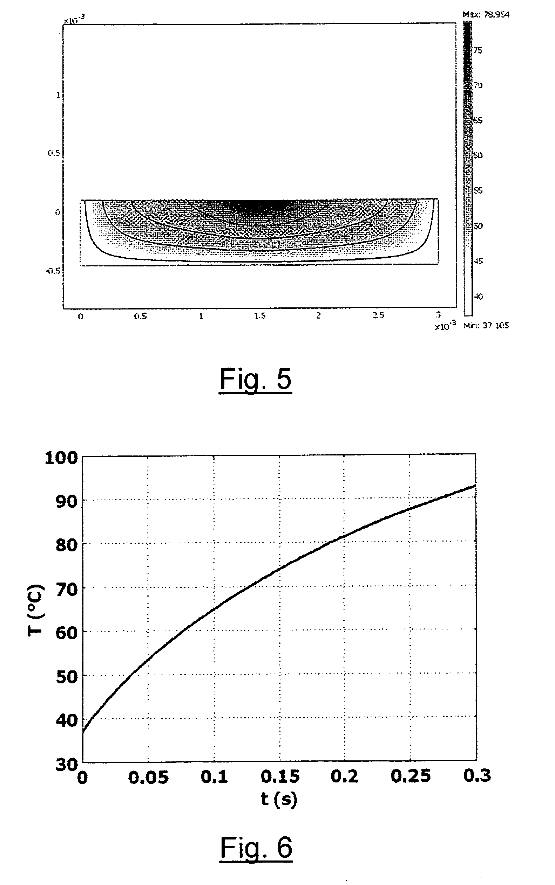

The following example shows an estimate of the increase in temperature and its distribution when the device is used in direct contact with the skin. For this purpose, it is important to bear in mind that the device dissipates heat during its operation; in particular, the above-mentioned LED dissipates a power of 8W. When applied in direct contact with the skin, we can assume that approximately 1W of this thermal power is transferred to the skin tissue by conduction. The dimensions of the area being illuminated coincides with that of the LED, i.e. a disc with a radius of 4mm. Assuming that the epidermis has been removed by dermal abrasion, the increase in temperature induced in the small-calibre superficial dermal capillaries can be calculated using the "bio-heat equation" and a finite element method (see, for instance, F. Rossi, R. Pini, "Modeling the temperature rise during diode laser welding of the cornea", in Ophthalmic Technologies XV, SPIE Vol. 5688, pp. 185-193, Bellingham, WA, USA, 2005). The resulting graph, as shown in figure 4, indicates the response of the temperature versus time in a capillary submitted to this type of treatment: we can see that, within a few seconds of irradiation (5-10 s), the temperature reached is capable of inducing the coagulation of the haematic component and thus stopping the bleeding. A map of the temperature distribution in the dermis, after 5 s of treatment, is given in figure 5. We can see a localised heating restricted to the volume being irradiated.

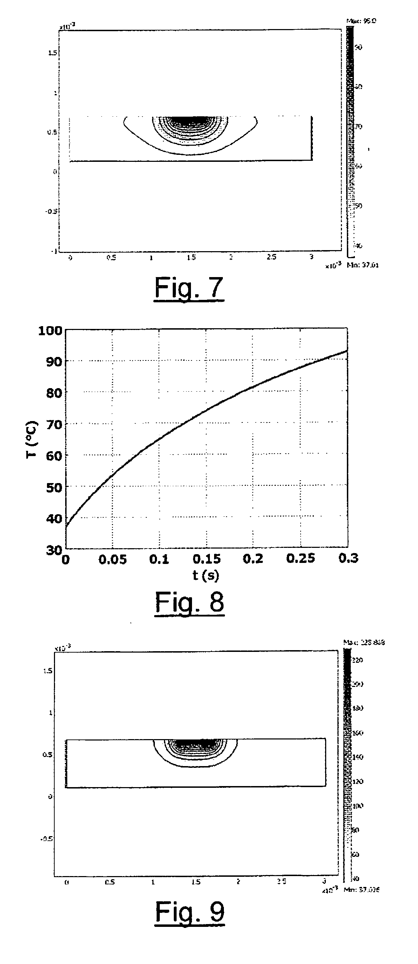

Application method with LED emission focusing. In another example, the same source - LED 435-66-60 (435 nm, 700 mW emission power) - is used but, in this case, the optical focusing system 7 is installed in front of the LED. This application method is suitable for the haemostasis of small skin areas of the order of a few square millimetres. Supposing that the focusing device gives rise to a loss of 50% of the power emitted by the LED, and that the radius of the illuminated area on the skin is 1 mm, the dynamics of the heating of the haematic component and the heat distribution in the tissue can be assessed. Figure 6 shows the calculated temperature response; it is observed that the desired heating effect is achieved after 0.3 seconds. The induced heat distribution using this consideration is rather localised, as shown in figure 7.

Application method using fibre-optic transmission of the light emitted by the LED. In another example, the same source - the LED435-66-60 (435 nm, 700 mW emission power) - is used but, in this case, in addition to the optical focusing system 7, the fibre-optic coupling system 9 is also installed. Assuming that a fibre with a radius of 0.5 mm is used, this method affords a very precise application of the treatment, that is more suitable for the haemostasis of small areas of skin, of the order of a few square millimetres. For instance, the method using fibre-optics is particularly advantageous for the haemostasis of bleeding caused during the dermal abrasion of individual wrinkles. Assuming that the fibre-optic coupling gives rise to a loss of 70% of the power emitted by the LED and that the radius of the area illuminated on the skin is 0.5 mm, we can evaluate the efficiency of the treatment in producing haemostasis, as in the previous cases. The results are given in figures 8 and 9. The desired heating effect is reached after 0.3 seconds and is extremely localised.

Application method with fibre-optic transmission and association of the light emitted by the LED with the light emission from a laser or high-power non-coherent light source. This method is used in the laser dermal ablation procedures, particularly when a short pulse erbium laser is used, that takes effect by means of a "cold ablation", i.e. it induces a negligible temperature increase in the skin, that is not enough to induce haemostasis. The operating precision achievable and the haemoglobin heating dynamics in the bleeding capillaries is comparable with that of the previous case.

LED modules comprise LEDs of different types, such as the above-mentioned LED 435-66-60-110 and the LED 405-66-60-110 (Roithner Lasertechnik, Vienna, Austria), to obtain light emissions coinciding with the absorption peaks of the oxygenated and non-oxygenated haemoglobin, respectively.

## Claims

1. A device for the haemostasis of cutaneous blood vessels, the device comprising LEDs (6), an electric power supply unit (2) for said LEDs and means for cooling (4) said LEDs and dissipating (5) the heat therefrom,
wherein said LEDs comprise two types of narrow-band LEDs emitting in different wavelength bands around 410 nm and in the range of 430 nm to 440 nm respectively.

2. A device according to claim 1, also comprising an optical focusing system (7) for said LEDs.

3. A device according to claim 2, also comprising a fibre-optic coupling system (10) for transmission of the focused light.

4. A system comprising a handpiece (15), a laser (16) or high-power non-coherent light source for surgical or cosmetic treatments and the device according to claim 3, **characterised in that** said device is coupled to the handpiece (15) through said fibre-optic coupling system (10).

5. A device according to any of claims 1 to 3, wherein the total output of the LEDs is comprised between 0.1 and 1 W.

## Patentansprüche

1. Vorrichtung für die Hämostase kutaner Blutgefäße, wobei die Vorrichtung LEDs (6), eine elektrische Leistungsversorgungseinheit (2) für die LEDs und Einrichtungen zum Kühlen (4) der LEDs und Abbauen (5) der Wärme davon enthält, wobei die LEDs zwei Arten von Schmalband-LEDs enthalten, die in verschiedenen Wellenlängenbändern um 410 nm bzw. im Bereich von 430 nm bis 440 nm emittieren.

2. Vorrichtung nach Anspruch 1, ebenfalls enthaltend ein optisches Fokussiersystem (7) für die LEDs.

3. Vorrichtung nach Anspruch 2, ebenfalls enthaltend ein Faser-Optik-Kopplungssystem (10) zur Transmission des fokussierten Lichts.

4. System enthaltend ein Handstück (15), einen Laser (16) oder eine nicht kohärente Hochleistungslichtquelle für Chirurgische oder kosmetische Behandlungen und die Vorrichtung nach Anspruch 3, **dadurch gekennzeichnet, dass** die Vorrichtung an das Handstück (15) durch das Faser-Optik-Kopplungssystem (10) gekoppelt ist.

5. Vorrichtung nach einem der Ansprüche 1 bis 3, wobei die gesamte Ausgabe der LEDs zwischen 0,1 und 1 W enthalten ist.

## Revendications

1. Un dispositif pour l'hémostase de vaisseaux sanguins cutanés, le dispositif comprenant des LEDs (6), une unité d'alimentation en énergie électrique (2) pour lesdites LEDs et des moyens pour refroidir (4) lesdites LEDs et en dissiper (5) la chaleur, dans lequel lesdites LEDs comprennent deux types de LEDs à bande étroite émettant respectivement dans des bandes de différentes longueurs d'ondes aux environs de 410nm, et entre 430nm et 440nm.

2. Un dispositif selon la revendication 1, comprenant en outre un système optique de focalisation (7) pour lesdites LEDs.

3. Un dispositif selon la revendication 2, comprenant en outre un système (10) de couplage par fibres optiques pour transmettre la lumière focalisée.

4. Un système comprenant une pièce à main (15), un laser (16) ou une source de lumière non-cohérente de forte énergie pour des traitements chirurgicaux ou cosmétiques et le dispositif selon la revendication 3, **caractérisé en ce que** ledit dispositif est couplé à la pièce à main (15) via ledit système de couplage par fibres optiques (10).

5. Un dispositif selon l'une quelconque des revendications 1 à 3, dans lequel la puissance totale de sortie des LEDs est comprise entre 0,1 et 1 W.
